# EUROPEAN PATENT APPLICATION

(11) **EP 4 197 522 A1**
(43) Date of publication of application: **21.06.2023**
(21) Application number: 21306864.6
(22) Date of filing: 20.12.2021
(51) Int. Cl.: A61K 8/60, A61Q 19/08, A61Q 17/00, A61Q 17/04

(54) **COMPOSITION COMPRISING GALACTINOL AND USE THEREOF**

(71) Applicant: Clariant International Ltd, 4132 Muttenz (CH)
(72) Inventor: CHAJRA, Hanane, 31170 TOURNEFEUILLE (FR); FRECHET, Mathilde, 31400 TOULOUSE (FR); Ter HALLE, Robert, 31450 BAZIEGE (FR); GUILLOTIN, Laure, 31400 TOULOUSE (FR); GONINDARD, Christophe, 31450 DONNEVILLE (FR)
(74) Representative: Mikulecky, Klaus

(57) **Abstract**

The invention relates to a composition comprising or consisting of 3-O-α-D-galactopyranosyl-D-myo-inositol as an active ingredient and to a process for biocatalytic production of a galactinol having the formula 3-O-α-D-galactopyranosyl-D-myo-inositol. Moreover, the instant invention describes the cosmetic use of a composition comprising or consisting of 3-O-α-D-galactopyranosyl-D-myo-inositol for maintaining and/or restoring a healthy skin through the stimulation of its own intrinsic defense mechanisms, for inducing the production of the extracellular matrix components in skin cells, for stimulating the growth factors production, for maintaining and/or reinforcing the skin microbiota; more generally for improving the global skin homeostasis and the global skin appearance .

## Description

### Field of the invention

The present invention relates to a composition comprising 3-O-α-D-galactopyranosyl-D-*myo*-inositol (galactinol) and its use as an active ingredient, particular in the cosmetic field, and to a method for preparing such composition. The present invention also pertains to the cosmetic use of 3-O-α-D-galactopyranosyl-D-*myo*-inositol as an active ingredient in a skin care composition.

### Background of the invention

At the interface between the organism and the environment, the skin - composed of three distinct layers: the stratum corneum (hereinafter called "SC"), the epidermis and the dermis - provides a mechanical protection and represents the first barrier against aggressions from the outside and against uncontrolled loss of body fluids. External aggressions are, for example, ultra-violet and infra-red exposure, particles matters' pollution, pathogens, allergens, fluctuation of temperature and humidity, chemicals products, mechanical constraints due to daily habits such as shaving, gloves, and mask wearing due to sanitary decline of our environment by the prevalence of harmful viruses. Additionally, the skin protects also the organism against internal aggressions such as chronological aging, hormonal fluctuation and immune deregulation. For example, intrinsic and extrinsic ageing processes are known to be associated with phenotypic changes in cutaneous cells, major structural and functional changes occur in the dermal extracellular matrix (ECM) that is a conjunctive tissue surrounding the cells, containing fibers and ground substances composed by water and macromolecules (e.g. proteoglycans). Indeed, fibrillar collagens, elastic fibers (e.g. elastin (ELN)) and proteoglycans are required to confer tensile strength, resilience (recoil), elasticity and hydration, respectively, to the skin. In this regard, collagens (COL) confer tensile strength to the skin. Collectively, glycoproteins and proteoglycans in conjunction with the abundant, polymeric hyaluronic acid (HA) are distributed throughout the skin where they perform a key role in maintaining skin hydration. Moreover, proteoglycans are present in many connective tissues on cell surfaces and in intracellular compartments.

It is known that proteoglycans such as decorin (DCN), perlecan (PLC), lumican (LUM), syndecan (SDC) and versican (VCAN), a family of glycosaminoglycan (GAG) conjugated proteins, are important constituents of human skin connective tissue (e.g. dermis) and are essential for maintaining mechanical strength of the skin (Martinez et al., "Role of proteoglycans on skin ageing: a review". International Journal of Cosmetic Science, Vol.42; p.529-535, 2020).

Indeed, these proteoglycans are essential components that restore the cells, providing hydration, maintaining hydration of the ECM, preventing the formation of wrinkles thanks to their ability to combine to other molecules such as collagen or hyaluronic acid and favoring the smoothness of the skin texture. For example, it is known that hyaluronic acid helps retain over 1,000 times its weight in water within the skin cells, making it an excellent moisturizer. Dermis contains up to 50% of body hyaluronic acid. Moreover, apart their hydrodynamic functions, proteoglycans have distinct biological functions. For example, decorin, which is found in dermis regulates collagen fibril formation and in modifying the activity of transforming growth factor-beta.

Moreover, all the aforementioned aggressions are known to induce many deleterious effects at cellular but also at the molecular level such as:
- the generation of reactive oxygen species (ROS). ROS provoke damages to multiple cellular organelles and processes, which ultimately disrupt normal cell physiology. Fortunately, a delicate balance exists between ROS production and the antioxidant defenses that protect cells *in vivo.* Indeed, cells have put in place various antioxidant defense mechanisms such as Nuclear Factor erythroid 2-related factor 2 (Nrf2) pathway, detoxifying enzymes (e.g. super oxide dismutase (SOD), glutathione peroxidase-1 (GPX1), NAD(H)dehydrogenase quinone 1 (NQO1) and catalase (CAT)) that are able to neutralize and/or metabolize these harmful ROS;
- proteome alteration through proteins denaturation, aggregation and misfolding, that could be counterbalanced by cell defenses such as heat shock proteins (HSP) and chaperone proteins involved in proteome integrity and repair. Particularly, the mammalian HSP90 family of proteins a cluster of highly conserved molecules involved in myriad of cellular processes are essential for cellular homeostasis. Moreover, HSP27 plays crucial roles on skin homeostasis as hydration, thanks to the regulation of the final steps of keratinization in stratum corneum. Moreover, HSP27 is described to be involved in filaggrin (FLG), loricrin (LOR) and keratin 10 (KRT10) production,
- DNA damages such as mutations, deamination, missing bases, chemical modification of bases, formation of pyrimidine dimers (e.g. Thymine dimers) and strand breaks could be counteracted by various DNA repair enzymes such as the growth arrest and DNA damage-inducible protein alpha (GADD45-α), the proliferating cell nuclear antigen (PCNA) produced by skin cells. DNA maintenance is known to be a crucial mechanism to limit senescence, aging, and apoptosis impact on cells and organs,
- the decrease in cell division rates (decrease in PCNA and Ki67 proliferative markers) in self-renewing tissues such as the skin leading to a reduced ability to maintain skin homeostasis.

Furthermore, when those aggressions become persistent and too excessive, they alter the capabilities of the skin to self-defend and to self-repair. Indeed, the skin is no longer able to induce its own intrinsic defense mechanisms such as its antioxidant machinery, its proteome protection thanks to the heat shock proteins, the DNA repair enzymes, the growth factors production but also to stimulate the production of its major ECM constituents. Consequently, the skin become more fragile, dehydrated, unhealthy and prone to a premature aging.

It is known that keratinocytes and fibroblasts are widely found in both the skin and the hair follicles.

Recently, the term "metaproteomic" appeared to describe the genes and/or proteins most abundantly expressed in environmental samples. More precisely, the term "metaproteomic" refers to a proteomic analysis of a mixture of species using an appropriate mass spectrometer (MS) to generate MS data and searching the MS data with a compilation of protein sequence databases that represent at least some of the species in the mixture. Exemplary method of metaproteomic analysis is known (Xiong Weili et al., Journal of Proteome Research., 14, 1, 133-141, 2014). Metaproteomics is a tool for the large-scale analysis of proteins in microbiomes that allows researchers to address a diversity of questions related to functions and interactions in microbiomes and host.

It is agreed that, cross-talks between host and its microbiome are an essential part of numerous biological functions, and the preservation of a correct balance between those two components of skin is essential for the human health.

Galactinol (3-O-α-D-galactopyranosyl-D-*myo*-inositol) is a natural molecule that is widely present among different plant species and is used as galactose donor for the synthesis of raffinose family of oligosaccharides (RFO's).

Galactinol is a disaccharide constituted by a myo-inositol bounded to a galactose. This nomenclature encompasses several isomers. Hence, there are multiple galactinol isomers depending on both the linkages between the two saccharides and the configuration of each saccharide as well.

The biosynthesis of galactinol from inositol and uridine diphosphate-galactose is known to be a key step in the synthesis of RFO's and is catalysed by the enzyme galactinol synthase (GoIS). In plant, the expression of GolS is induced by abiotic stress and triggers as such the synthesis of RFO's as a mean to protect the plant against drought and other types of stress such as salinity and cold.

In plant, galactinol is present in a very small amount and its production in sufficient amount for satisfying the needs in cosmetic domain is challenging. For example, a small amount of galactinol can be isolated from sugar beet sirup or from castor oilseed meal (McCready et al., Journal of the A.S.S.B.T: 127-132, 1965). The galactinol isomer (1-O-α-D-galactopyranosyl-*myo*-inositol) is also obtainable from biocatalytic synthesis from three substrates (sucrose, uridine diphosphate (UDP) and myo-inositol) by using three enzymes (sucrose synthase, UDP-galactose-4-epimerase and galactinol synthase) (Wakiuchi et al., Bioscience, biotechnology and biochemistry 67 (7): 1465-1471, 2003). Using this biocatalytic synthesis, a single product is obtained in which *myo*-inositol is selectively galactosylated to obtain a product that is identical to galactinol that has been isolated from soya beans (Schweizer et al., Carbohydrates Research, 95, 61-71, 1981).

Galactosylation in general can be performed by transglycosylation in which a galactose donor such as melibiose is used for, for example, the galactosylation of tyrosol (Potockà et al., Journal of Molecular Catalysis B: Enzymatic 113, 23-28, 2015).

The synthesis of galactinol can be applied to the biocatalytic synthesis of soybean oligosaccharides such as raffinose and stachyose as described in the document CN-A 107586809. It describes that raffinose synthetase and stachyose synthetase that catalyse the conversion of sucrose to raffinose and raffinose to stachyose respectively are being exploited using galactinol as galactose donor. However, this biocatalytic synthesis needs in total three different enzymes to run the whole synthesis of galactinol.

Galactinol isomers are already used in cosmetic field.

For example, JP-A 2007084484 describes inositol glycosides, including *myo-*inositol bounded to galactose (galactinol) for alleviating cell damage caused by exposure to UV-rays, thus in preventing the skin pigmentation. Among the galactinol isomers, are disclosed the β-isomers. Are also described the galactinol isomers having the linkages 1-O-α-D, 2-O-α-D, 4-O-α-D and 5-O-α-D-galactopyranosyl-*myo*-inositol. However, the 3-O-α-D-galactopyranosyl-D-*myo-*inositol is missing.

The document US-B2 10918585 teaches a skin anti-aging composition consisting of inositol derivatives in which glucose or an oligosaccharide comprising glucose as a constituent unit is bounded to *myo*-inositol. However, galactose is not disclosed as oligosaccharide.

The aforementioned inositol derivatives are also used as agent for inhibiting skin trouble caused by fine particulates matters (e.g. EP-A1 3636266).

The document EP-B1 1744725 discloses *myo*-inositol bounded with glucose for improving the skin moisture.

The document JP-B2 4624831 describes a skin external preparation comprising an inositol bounded to a sugar for improving the skin moisture and smoothness. Galactose is not disclosed as a sugar.

The aforementioned galactinol isomers have several technical drawbacks. Often, their production by biosynthesis is costly as it implies several enzymes and substrates in multiple enzymatic cascades, generating a huge amount of unusable by-products. Furthermore, each of those enzymes and substrates can be expensive, leading unquestionably to a high cost production of the final product (galactinol), which itself is becoming expensive. Another technical drawback is that all enzymes disclosed for the biocatalytic synthesis of galactinol are plant-derived enzymes and as such can sometimes be difficult to express in prokaryotic expression hosts. Lastly, to the best of our knowledge, the production of galactinol with high yields is not satisfying to reply to the cosmetic needs.

Therefore, there is still an unmet need to produce galactinol for use in cosmetic field. Moreover, it is desired that the galactinol is from natural origin.

There is also still an unmet need for further efficient biocatalytic synthesis for producing, at a lower cost, a natural galactinol isomer by using melibiose or another substrate as galactose donor. Furthermore, hence the yield of galactinol production by known biocatalytic synthesis is low, there is still an unmet need in providing in a large-scale production method of galactinol with a limited amount of its isomers and/or its isoforms.

It is also desired to keep a healthy skin by protecting it against harmful factors. Furthermore, it is also sought having skin presenting good physiological and physical properties, thus having global good appearance.

These objectives may be reached by the means of a composition of natural origin capable to mobilize and/or activate, in healthy skin, its own intrinsic defense mechanisms (e.g. antioxidant machinery, protection of its proteome, DNA repair enzymes, production of growth factors) and to preserve its microbiota ressources. Consequently, the skin can become more remodelled, hydrated, protected, healthy, more resilient to both the premature aging and pro-aging processes, and more generally the skin is presenting a youthful and healthier global appearance.

### Summary of the invention

Surprisingly, it has been found that a "natural" galactinol compound corresponding to the formula 3-O-α-D-galactopyranosyl-D-*myo*-inositol can be obtained by the means of a new biocatalytic synthesis. As a result, it was also found that *myo-*inositol can be efficiently galactosylated with a high yield.

The present invention relates to a composition comprising 3-O-α-D-galactopyranosyl-D-*myo*-inositol obtainable by a biocatalytic synthesis starting with a melibiose as galactose donor.

Accordingly, an aspect of the present invention relates to a composition comprising 3-O-α-D-galactopyranosyl-D-*myo*-inositol as an active ingredient.

Another aspect of the present invention relates to a process for biocatalytic production of a galactinol having the formula 3-O-α-D-galactopyranosyl-D-*myo-*inositol comprising the steps:
a.1) Optionally providing melibiose, or incubating raffinose with β-fructofuranosidase, at pH of 3 to 8, for obtaining melibiose; and incubating the mixture comprising melibiose and *myo*-inositol with α-galactosidase at pH of4 to 9; or
a.2) Incubating a mixture comprising *myo*-inositol and raffinose and/or stachyose with α-galactosidase at pH of 4 to 9; without having conducted step a1);
b. Purifying 3-O-α-D-galactopyranosyl-D-*myo*-inositol from the mixture of compounds obtained in step a1) or in step a2).

Another aspect of the present invention refers to a cosmetic or pharmaceutic composition comprising:
a. 3-O-α-D-galactopyranosyl-D-*myo*-inositol as an active ingredient; and
b. at least one further cosmetically and/or pharmaceutically acceptable ingredient other than 3-O-α-D-galactopyranosyl-D-*myo*-inositol,
wherein the composition is a composition for topical use selected from the group consisting of a solution, a suspension, an emulsion, a cream, a paste, a gel, a lotion, a powder, a soap, a surfactant-containing water, an oil, a shampooing, and a spray; and/or wherein the composition is a nutraceutical composition which is administered orally.

Another aspect of the present invention refers to a cosmetic use of a composition comprising 3-O-α-D-galactopyranosyl-D-*myo*-inositol for maintaining and/or restoring a healthy skin through the stimulation of its own intrinsic defense mechanisms, for inducing the production of the extracellular matrix components in skin cells, for stimulating the growth factors production; more generally for improving the global skin homeostasis and the global skin appearance.

A further aspect of the present invention refers to a composition comprising (or consisting of) 3-O-α-D-galactopyranosyl-D-*myo*-inositol as an active ingredient for use in a non-therapeutic method for maintaining and/or for improving the global appearance of the skin and its annexes, wherein the composition is for maintaining or ameliorating the skin homeostasis, for maintaining a healthy skin, for improving the skin firmness and/or elasticity, for protecting skin against external stress factors, for improving skin relief, for improving the skin hydration and skin barrier function, for preventing and/or delaying the signs of skin aging; and wherein, for already aged skin, the composition is for preserving and reinforcing the mobilization of its microbiota resources.

The experimental section of the present application demonstrates that the composition of the present invention is usable for improving the global appearance of the skin and its annexes. It may plausibly upregulate the expression of the main genes, key proteins, proteoglycans and glycosaminoglycans involved for accomplishing this objective.

### Brief description of Figures

Figure 1 shows the chemical structure of galactinol according to the invention.
Figure 2 shows the ability of the galactinol and a composition comprising thereof according to the embodiment of the invention to stimulate in normal human dermal fibroblasts (NHDF) the gene expression of extracellular matrix (ECM) components. The set of the modulated genes comprises collagen type V alpha 1 chain (COL5A1); Elastin (ELN), Laminin Subunit Gamma 2 (LAMC2), Lumican (LUM), Syndecan-4 (SDC4), Versican core protein (VCAN).
Figure 3 shows the ability of the galactinol and a composition comprising thereof according to the embodiment of the invention to stimulate in NHDF the antioxidant enzymes. The set of modulated genes comprises, NAD(H)dehydrogenase quinone 1 (NQO1), heme oxygenase 1 (HMOX1), glutathione peroxidase 1 (GPX1) and glucose-6phosphate dehydrogenase (G6PD).

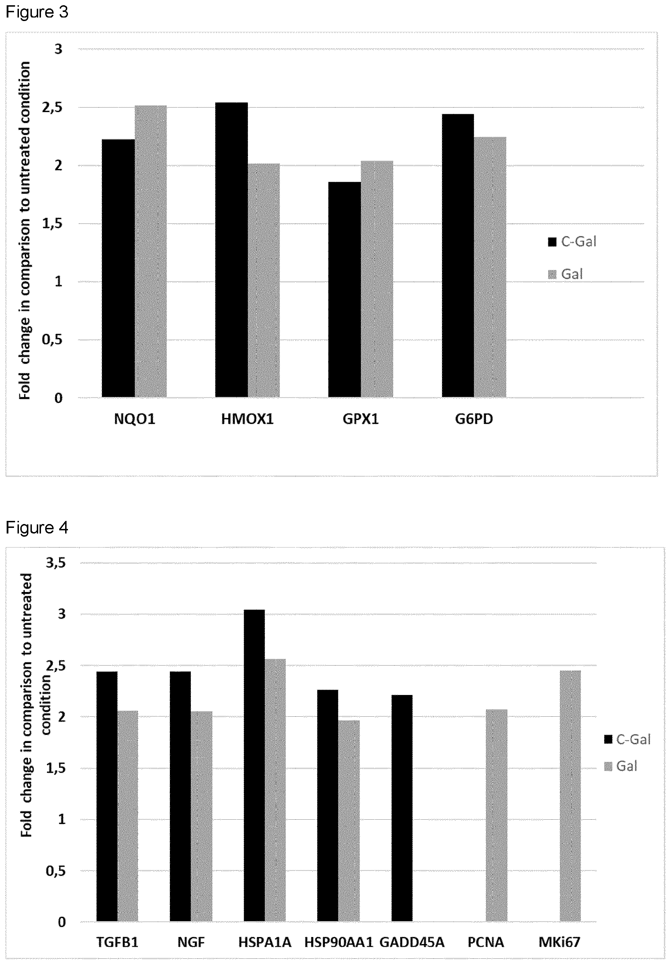

Figure 4 demonstrates the ability of the galactinol and a composition comprising thereof according to the embodiment of the invention to stimulate in NHDF the mRNA expression of major genes involved in the maintenance of global skin homeostasis, namely protein's protection (heat shock proteins such as heat shock protein Family A member A (HSPA1A) and heat shock protein 90 alpha family class A (HSP90AA); DNA repair and cell proliferation: proliferating cell nuclear antigen (PCNA), marker of proliferation Ki-67 (MKi67) and growth arrest and DNA damage 45-alpha (GADD45A); and growth factors such as transforming growth factor beta-1 (TGFβ1) and nerve growth factor (NGF).
Figure 5 demonstrates the ability of the galactinol and a composition comprising thereof according to the embodiment of the invention to protect the NHDF's proteome by the stimulation in these cells of an important expression of HSP27.
Figure 6 shows the ability of the composition comprising galactinol according to the invention to stimulate in NHEK the production of hyaluronic acid (HA).
Figure 7 shows at the clinical level, on human volunteers the ability of the composition comprising galactinol according to the invention to improve skin biomechanical properties (by increasing Ue and Ur parameters), to reduce skin wrinkles and to attenuate skin microrelief (smoothing effect).

### Detailed description of the invention

An aspect of the present invention relates to a composition comprising (or consisting of) 3-O-α-D-galactopyranosyl-D-*myo*-inositol as an active ingredient.

It will be understood that, in a composition consisting of 3-O-α-D-galactopyranosyl-D-*myo*-inositol, the composition is 3-O-α-D-galactopyranosyl-D-*myo*-inositol.

As used throughout the present disclosure, the 3-O-α-D-galactopyranosyl-D-*myo-*inositol of the present invention is also designated as "galactinol" [Cas number: 3687-64-7].

In an embodiment, the composition of the present invention may comprise a galactinol isomer having α-linkage other than the 3-α-linkage. Advantageously, the galactinol isomer having α-linkage other than the 3-α-D may be selected from 1-O-α-D-, 2-O-α-D-, 4-O-α-D-, 5-O-α-D, or 6-O-α-D-galactopyranosyl-D-*myo-*inositol. The molar ratio between the galactinol:galactinol isomer within the composition of the invention may be comprised approximatively at 3:1, preferably 2:1, in particular 1.5:1.

In a preferred embodiment, the mixture comprising the galactinol and the said galactinol isomer represents 20% by weight, preferably 25% by weight, in particular 30% by weight relative to the total weight of the composition.

In a preferred embodiment, the composition of the present invention may further comprise melibiose, *myo*-inositol, monosaccharide, or a mixture thereof, wherein monosaccharide being selected from galactose, glucose, fructose, or a mixture thereof.

In a preferred embodiment, the composition of the present invention may further comprise, referred to the total weight of the composition:
- melibiose between 0 to 25%, preferably 0,1 to 20% by weight,
- myo-inositol between 0 to 40%, preferably 0,1 to 35% by weight,
- monosaccharides between 0 to 30%, preferably 0,1 to 25% by weight.

Advantageously, the composition of the present invention may comprise the galactinol as described above, for example in mixture with one or more of the aforementioned compounds. Preferably, the relative amount of each of these compounds within the composition of the invention may be in the ranges as described above.

A process for biocatalytic production of a galactinol having the formula 3-O-α-D-galactopyranosyl-D-*myo*-inositol comprising the steps:
A1) Providing melibiose, or incubating raffinose with β-fructofuranosidase, at pH of 3 to 8, for obtaining melibiose; and incubating the mixture comprising melibiose and an acceptor selected from pinitol and/or inotisol isomers comprising *myo-, chiro-, scyllo-, chiro-, neo-, allo-, epi-* and *cis*-inositol, preferably *myo*-inositol with α-galactosidase at pH of 4 to 9; or
A2) Incubating a mixture comprising an acceptor selected from pinitol and/or inotisol isomers comprising *myo-, chiro-, scyllo-, chiro-, neo-, allo-, epi-* and *cis*-inositol, preferably *myo*-inositol and raffinose and/or stachyose with α-galactosidase at pH of 3 to 9; without having conducted step A1);
B) Purifying 3-O-α-D-galactopyranosyl-D-*myo*-inositol from the mixture of compounds obtained in step A1) or in step A2).

Hence, the present invention aims to provide a process using an abundant galactose donor, by using an enzyme capable to perform an α-transglycosylation mainly in position 3 of the *myo*-inositol.

Advantageously, raffinose is a natural compound which is abundant in nature and cheap. For example, raffinose may be isolated from plants. Preferably, raffinose may advantageously be isolated, but not exclusively, from cottonseeds, soybeans seeds, or leguminous such as cabbage, broccoli, or asparagus.

It is worth noting that raffinose, melibiose and *myo*-inositol may be in solid form (e.g. powder) before the incubation step with the enzyme. Therefore, the said substrates donors may be solubilized within a suitable solvent and/or buffer during incubating with the enzyme. Accordingly, in an embodiment, raffinose may be dissolved with a buffer prior the incubation step with β-fructofuranosidase. In a preferred embodiment, raffinose is dissolved with sodium phosphate buffer at pH from 4 to 8, preferably pH is in the range of 4.5 to 7, more preferably pH is 4.9 to 6.5.

As used herein, "β-fructofuranosidase" also called invertase, refers not only to enzymes of the EC 3.2.1.26 class but also to molecules or functional equivalents thereof which exhibit the same properties in relation to its substrates and products. "Functional equivalents" or analogs of the enzymes as used herein are various polypeptides thereof, which moreover process the desired biological function and/or activity, such as enzyme activity. Advantageously, it is an enzyme of yeast origin capable to specifically hydrolyzing the terminal non-reducing β-D-fructofuranoside residues in β-D-fructofuranosides.

As used herein, the term "a-galactosidases" also called melibiases, refers not only to enzymes of the EC 3.2.1.22 class but also to molecules or functional equivalents thereof which exhibit the same properties in relation to its substrates and products. Advantageously, these enzymes of microbial origin capable to specifically hydrolyzing the terminal, non-reducing α-D-galactosyl moieties from a wide variety of products such as glycoproteins, glycolipids or oligosaccharides including galactose oligosaccharides, galactomannans and galactolipids. Apart from these canonical reactions, these enzymes can also catalyse the formation of an α-galactosyl bond. This can be done in either a "reverse hydrolysis" manner or by using an activated galactose donor. In the first case, an equilibrium is reached between the reagents (galactose and acceptor) and the products (galactosylated product and water). The position of this equilibrium lies on the left-hand side and often high concentrations of galactose are required to obtain low conversions.

According to the invention, preferred acceptor is *myo*-inositol. Other inositol isomers such as *chiro-, scyllo-, chiro-, neo-, allo-, epi-* and *cis*-, or inositol derivatives such as pinitol could be used as they also act as acceptor.

In a preferred embodiment the composition comprises
- melibiose 0 to 25 weight %,
- *myo*-inositol 0 to 40 weight %,
- monosaccharides 0 to 30 weight %
over the total weight of the composition.

Using an activated galactose donor in a transglycosylation reaction may allow to obtain higher conversions and typical α-galactose donors are p-nitro-phenol-α-galactose and melibiose. The latter has the advantage of being a relatively abundant natural molecule. However, although melibiose is commercially available, it is quite expensive, compared to raffinose for example. Raffinose and stachyose can be used as well as galactose donor but give lower yield of galactinol.

As used herein, the term "melibiose" or 6-O-α-D-galactopyranosyl-D-glucose, is a reducing disaccharide of plant origin. Advantageously, melibiose may be isolated for example, from Malvaceae families, coffee seeds, or apple. Melibiose can be commercially available, in different form, for example like a powder. However, to date, it is known that melibiose is quite expensive. Processes for production of melibiose are known in the art.

The β-fructofuranosidase and the α-galactosidase, or both may be employed in the incubation step as either a cell-free enzyme, which may need not be partially purified, a whole-cell system pre-treated physically or chemically for improved permeability of the cell membrane (permeabilization) and mechanical stability, encapsulated catalyst in which said free enzyme or whole-cell system are entrapped, or immobilized on a carrier, such a solid support (e.g. chromatography resin).

Preferably, the β-fructofuranosidase and the α-galactosidase, or both are for example natively or recombinant expressed in a microorganism. For example, native enzymes may be produced by fungi such as Aspergillus families, yeast such as Saccharomyces families. For example, recombinant enzymes may be produced in expressing hosts such as *Escherichia coli* and *Bacillus subtilis,* or *Pichia pastoris.*

In an embodiment, the β-fructofuranosidase and the α-galactosidase or both are for example a native form or genetically modified forms, e.g. resulting in an increased activity of the β-fructofuranosidase and the α-galactosidase, or both. Preferably, the α-galactosidase may be in its native form.

In a preferred embodiment, the fungi producing α-galactosidase may be, for example, *Aspergillus niger.* The yeast producing β-fructofuranosidase, may be, for example, *Saccharomyces cerevisiae.*

In another embodiment, the incubation pH of α-galactosidase is in the range from 3 to 9. In a preferred embodiment, the incubation pH is from 3.5 to 8.5. In a more preferred embodiment, the incubation pH is from 3.5 to 8.

In an embodiment, the incubation temperature of α-galactosidase may be comprised at 20 to 70°C, preferably at 25 to 65°C, more preferably at 30 to 60°C, even more preferably at 35 to 55°C, in particular at 40 to 50°C.

In an embodiment, the β-fructofuranosidase and the α-galactosidase are for example either added separately or at the same time to the raffinose or the mixture of melibiose and *myo*-inositol.

In a preferred embodiment, β-fructofuranosidase is added to raffinose, for example for 30 to 150 minutes at 20 to 60°C, preferably at 25 to 55°C, in particular at 30 to 50°C for allowing the conversion of raffinose into melibiose and fructose.

In an embodiment, the incubation pH of β-fructofuranosidase is in the range from 3 to 8. In a preferred embodiment, the incubation pH is from 4 to 7.

In an embodiment, α-galactosidase may be added, either for example, to the melibiose in the step a) in which *myo*-inositol is added afterwards, or to a mixture comprising melibiose and *myo*-inositol. Preferably, *myo*-inositol may be, for example, added at the end of the reaction in step a) where raffinose is quantitatively converted into melibiose and fructose. More preferably, *myo*-inositol may be added after 30 to 150 minutes after the step A1) was being started. Advantageously, the enzymatic reaction may be stopped by inactivating the enzyme. For example, α-galactosidase may be stopped by increasing the temperature approximatively at 65 to 80°C, preferably at 72 to 78°C for 1 to 10 minutes, preferably for 3 to 6 minutes.

In a preferred embodiment, incubating a mixture comprising *myo*-inositol and raffinose and/or stachyose with α-galactosidase at pH from 3 to 9, preferably at pH from 3.5 to 8,5, in particular at pH from 3.5 to 8, allows to obtain galactinol. In this case, conducting the step A1) is not needed. Advantageously, both raffinose and stachyose may be used as both are acting as galactose donor.

The reaction mixture containing galactinol obtained in step A1) or in step A2) may be purified by the means of purification methods known in the art. Purification methods may include separation through an ultrafiltration membrane with a constant molecular weight cut-off value, or separation through various chromatographic methods, or one or more of the followings methods: liquid-solid separation, fermentation, crystallization, and precipitation.

Accordingly, as a preferred purification step of the invention, it may be performed a fermentation treatment, for example by the means of fungi such as yeasts (e.g., Baker's yeast). Such purification by fermentation using fungi is known (McCready et al., Journal of the A.S.S.B.T: 127-132, 1965). Advantageously, since melibiose, galactinol and its isomer and *myo*-inositol are non-fermentable compounds, they are not affected by the yeast fermentation. In other words, they remain in the mixture obtained in step A1) and in step A2). In contrary, as the monosaccharides such as glucose, fructose and galactose are fermentable, the yeast fermentation allows to consume mainly these monosaccharides from the mixture.

In an embodiment, the purification of the reaction mixtures obtained A1) and in step A2) may be conducted by yeast fermentation. The yeast fermentation may be carried out for a suitable time for allowing the total consumption of fermentable monosaccharides. Advantageously, the yeast fermentation may be performed for 5 to 60 hours, preferably for 10 to 50 hours, in particular for 15 to 45 hours at 20 to 40°C, preferably approximatively at 25 to 35°C.

Additional purification step may be carried out after the yeast fermentation described above.

In a preferred embodiment, a precipitation step by the means of hydroalcoholic solvent may be performed in the remaining reaction mixture obtained after the yeast fermentation. Advantageously, the said remaining reaction mixture may comprise essentially galactinol, and also melibiose and myo-inositol. If this latter is present in the remaining reaction mixture, it can efficiently be removed, at least partly or totally, for example by filtration after the precipitation step followed by a solvent elimination, which may be done, for example by solvent evaporation.

In a preferred embodiment, the remaining reaction mixture may be decanted or preferably centrifuged after the yeast fermentation. A solvent evaporation may be conducted on the supernatant at 40 to 80°C, preferably at 50 to 75°C, more preferably at 60 to 70°C under reduced pressure. The concentrated remaining reaction mixture obtained may be cooled at 15 to 0°C, preferably at 2 to 5°C. The simultaneous cooling and the addition of a hydroalcoholic solvent, preferably hydroethanolic, improves the precipitation yield, thus the formation of the crystals. At the end of this step, anhydrate crystals, essentially constituting by *myo*-inositol, are obtained. These crystals may be washed with an alcohol (e.g., ethanol) and filtered off. Advantageously, the said myo-inositol crystals, that could be considered as by-product, can advantageously be reused for another biocatalytic production of galactinol thereby increasing the atom-efficiency of such subsequent process.

Accordingly, the hydroalcoholic solvent may be any solvent mixture that comprises at least one alcohol. Preferably, the hydroalcoholic solvent is mainly composed of one or more alcohols and water. Preferably, the hydroalcoholic solvent is a solvent mixture that is composed of more than 20%, at least 50%, at least 70%, at least 80%, at least 90%, at least 99% by weight, consists (essentially) of one or more alcohols and water. As used herein, water may also include hot water or subcritical water. Also, one or more aqueous buffers may be used.

As used herein, the term "hydroalcoholic" may be understood in the broadest sense as generally understood in the art. It may be any composition comprising alcohol and water. A hydroalcoholic solvent may be any alcohol/water mixture. In a preferred embodiment, the solvent is an alcohol/water mixture containing between 50 and 99% (v/v) alcohol, or containing between 60 and 99% (v/v) alcohol, or containing between 65 and 99% (v/v) alcohol, or containing between 70 and 99% (v/v) alcohol, or containing between 75 and 98% (v/v) alcohol, or containing between 80 and 98% (v/v) alcohol, or containing approximately 97% (v/v) alcohol.

In a preferred embodiment, the alcohol may be selected from the group consisting of methanol, ethanol, propanol, butanol, pentanol, phenol, propanediol, glycerol, and a mixture of two or more thereof.

In a preferred embodiment, the hydroalcoholic solvent may be selected from a mixture of water/ethanol, water/methanol, and water/propanol.

In a preferred embodiment, a hydroalcoholic solvent may be a hydroethanolic solvent. Thus, in a preferred embodiment, the solvent is an ethanol/water mixture containing between 50 and 99% (v/v) ethanol, or containing between 60 and 99% (v/v) ethanol, or containing between 65 and 99% (v/v) ethanol, or containing between 70 and 99% (v/v) ethanol, or containing between 75 and 98% (v/v) alcohol or containing between 80 and 98% (v/v) alcohol, or containing approximately 97% (v/v) ethanol. In another preferred embodiment, a hydroalcoholic solvent may be a hydromethanolic solvent.

In an embodiment, the galactinol may be purified from the reaction mixture after the precipitation step described above. Advantageously, for example, the purification step for obtaining galactinol may be conducted by the means of chromatography by affinity, such as activated carbon column. Such purification method consisting to absorb the reaction mixture on a column followed by successive elution by solvents is known in the art.

In an embodiment of the invention, after having absorbed the reaction mixture on the column, it may be eluted with, for example, a polar solvent. A suitable polar solvent may be a mixture of water and a primary alcohol having C₁ to C₅ carbon atoms. The primary alcohol may be selected from methanol, ethanol, propanol, butanol and pentanol. Preferably, the alcohol may be selected from methanol and ethanol, in particular ethanol.

In an embodiment of the invention, the amount of the alcohol within the hydroalcoholic solvent may be comprised between 1 to 20% (v/v) alcohol, preferably between 2 to 15% (v/v) alcohol, in particular between 3 to 10% (v/v) alcohol. The alcohol may be selected from methanol, ethanol, propanol, butanol and pentanol. Methanol and ethanol are preferable, and ethanol may be the most preferable one.

In a preferred embodiment of the invention, the elution with hydroalcoholic solvent may be conducted sequentially with an increasing alcohol amount starting from 1%, preferably from 2% (v/v) of alcohol, in particular from 3% (v/v) of alcohol and ending to 20% (v/v) of alcohol, preferably to 15% (v/v) of alcohol, in particular to 10% (v/v) of alcohol. Accordingly, elution steps may comprise two or more sequences, preferably three sequences.

In an embodiment of the invention, the last fractions eluted with 10 to 20% (v/v) of alcohol may be combined and concentrated under reduced pressure for at least partly evaporating, or totally evaporating solvent, till dryness for obtaining a dried matter (in physical form).

In a preferred embodiment of the invention, the said dried matter may be solubilized in a water and subsequently purified by chromatography by affinity, such an activated carbon as described above. This second purification may be conducted identical to the aforementioned first one. Two fractions may be collected in that consisting of galactinol and galactinol isomer having α-linkage other than the 3-α- may be selected from 1-O-α-D-, 2-O-α-D-, 4-O-α-D-, or 5-O-α-D-, or 6-O-α-D-galactopyranosyl-D-*myo*-inositol.

In a preferred embodiment of the invention, both the galactinol and the said galactinol isomer may have a purity between 60 to 100%, preferably between 65 to 99%, more preferably between 70 to 98%.

Optionally, after the aforementioned precipitation step, a decoloration step may be conducted on the remaining mixture from which myo-inositol crystals have essentially removed. For example, the decoloration may be conducted by using hydrophobic resin.

As the method for biocatalytic production of a compound is known in the art, the person skilled in the art may select the best conditions for conducting the process of the present invention, namely in terms of temperature, concentration of enzymes, pH, duration, solvents, etc. by referring to the supplier's enzyme information.

The process according to the present invention may be performed in an industrial scale. Industrial production of galactinol of the present invention for example takes place in reactor well known in the art. For example, the process can be conducted according to a batch process, fed-batch process, continuous process, or combination thereof. The reactor size is not critical.

Accordingly, the present invention also refers to a composition comprising 3-O-α-D-galactopyranosyl-D-*myo*-inositol obtainable (or obtained) from the biocatalytic method described above.

Another aspect of the present invention refers to a cosmetic or pharmaceutic composition comprising:
a. 3-O-α-D-galactopyranosyl-D-*myo*-inositol as an active ingredient; and
b. at least one further cosmetically and/or pharmaceutically acceptable ingredient other than 3-O-α-D-galactopyranosyl-D-*myo*-inositol,
wherein the composition is a composition for topical use selected from the group consisting of a solution, a suspension, an emulsion, a cream, a paste, a gel, a lotion, a powder, a soap, a surfactant-containing water, an oil, a shampooing, and a spray; and/or wherein the composition is a nutraceutical composition which is administered orally.

It will be understood that the definitions and preferred embodiments made in the context of the galactinol of the present invention *mutatis mutandis* apply to a composition comprising thereof.

Herein, 3-O-α-D-galactopyranosyl-D-*myo*-inositol may optionally be considered as an (or optionally even the sole) active ingredient. The term "active ingredient" may be understood in the broadest sense as a component that may exhibit a desired and intended activity, either alone or together with one or more other ingredients such as one or more carriers that are themselves inactive or other active ingredient that may optionally act synergistically. Preferably, the active ingredient of the present invention is a cosmetically active ingredient.

The cosmetic or pharmaceutic composition may be used for any purpose and in any form. In a preferred embodiment, the composition is a composition for topic use selected from the group consisting of a solution, a suspension, an emulsion, a cream, a paste, a gel, a lotion, a powder, a soap, a surfactant-containing water, an oil, a shampooing, and a spray, or wherein the composition is a nutraceutical composition which is administered orally.

The composition may also be or may be comprised in a product selected from the group consisting of emulsions, gels, ointments, tonics, liquid soaps, bar soaps, bath oils, shower oils, massage oils, makeups, scalp treatments, aftershaves, shaving products, deodorants, shower gel, shampoos, and combinations of two or more thereof.

Optionally a cosmetic or pharmaceutical composition may also be a nutraceutical composition which can be administered orally to a subject. Then, the cosmetic or pharmaceutical composition may optionally be included in a food product, such as a food supplement. Then, the composition may typically have a systemic effect. A nutraceutical composition according to the present invention may be formulated, with acceptable carriers, in any form suitable for oral administration such as, e.g., tablets, capsules, granules, powder, solution, emulsion, or suspension.

In an embodiment, the composition may comprise less than 20% by weight, of galactinol. In a preferred embodiment, the composition comprises 0.0001 to 15% by weight, more preferably 0.001 to 10% by weight, even more preferably 0.005 to 8% by weight, in particular 0.01 to 5% by weight of galactinol.

The at least one further cosmetically and/or pharmaceutically acceptable ingredient other than galactinol may be any cosmetically and/or pharmaceutically acceptable ingredient. In a preferred embodiment, the cosmetically and/or pharmaceutically acceptable ingredient is or comprises at least one cosmetically and/or pharmaceutically acceptable carrier.

As used herein, the terms "pharmaceutically acceptable carrier", "pharmaceutically acceptable excipient", "cosmetically acceptable carrier", "cosmetically acceptable excipient", "carrier" and "excipient" may be understood interchangeably in the broadest sense as any substance that may support the cosmetic and/or pharmacological acceptance or usability of the composition according to the present invention containing galactinol.

The composition ready to use preferably may be a liquid formulation, in particular a composition suitable for topic administration. The storage form of the composition may also be liquid but may also be a dried form (e.g. a powder such as a powder comprising or consisting of galactinol or may be a paste or syrup or the like. Optionally, a dried form, paste or syrup may be dissolved or emulsified prior to being administered to the skin of interest.

A cosmetically and/or pharmaceutically acceptable carrier may exemplarily be selected from the list consisting of an aqueous buffer, saline, water, alcohols, vegetable oils, mineral oils, polymers, or combination of two or more thereof.

Optionally, a cosmetically and/or pharmaceutically acceptable carrier may contain one or more cosmetically and/or pharmaceutically acceptable additives. In a preferred embodiment, such cosmetically and/or pharmaceutically acceptable additives may be selected from the group consisting of fragrances/perfumes, dyes, pigments, emulsifiers, lubricants, chelating agents, acidity regulators, antimicrobial agents, preservatives, antioxidants, and combinations of two or more thereof. For instance, the cosmetically and/or pharmaceutically acceptable carrier may optionally contain one or more detergents, triethanolamine, one or more fragrances, one or more foaming agents (e.g., sodium lauryl sulphate (SLS), sodium doceyl sulphate (SDS)), one or more colouring agents (e.g., food colouring, pigments), one or more vitamins, one or more salts (e.g., sodium, potassium, calcium, zinc salts), one or more humectants (e.g., sorbitol, glycerol, butylene glycol, propylene glycol, mannitol, propylene glycol, polydextrose), one or more enzymes, one or more preserving agents (e.g., benzoic acid, methylparabene, one or more antioxidants, one or more herbal and plant extracts, one or more stabilizing agents, one or more chelating agents (e.g., ethylenediaminetetraacetic acid (EDTA), one or more polymers (e.g., carboxyvinyl polymer, carboxymethyl cellulose, cellulose, carboxyethyl cellulose), one or more stabilizers, one or more solubilizers, one or more emollients, and/or one or more uptake mediators (e.g., polyethylene imine (PEI), a cell-penetrating peptide (CPP), a protein transduction domain (PTD), an antimicrobial peptide, etc.). Moreover, the composition of the present invention may contain, in addition to 3-O-α-D-galactopyranosyl-D-myo-inositol, one or more other active ingredients, working either synergistically and/or complementarily with the said 3-O-α-D-galactopyranosyl-D-myo-inositol without affecting its activity or its indented use as mentioned above.

The composition according to the present invention may be a cosmetic product or may be comprised in a cosmetic product. Deleterious effects in the skin may be reduced or avoided, according to the safety studies. Any administration routes are suitable that lead to the desired purpose as claimed. Administration may be local or systemic administration. Preferably, administration is local administration. Administration may be topic administration, transdermal administration, oral administration, administration by mean of injection (e.g., intravenous (i.v.), intraarterial (i.a.), intraperitoneal (i.p.), intramuscular (i.m.), and subcutaneous (s.c.) injection), or nasal administration. For example, the composition according to the present invention may be formulated, with acceptable carriers in any suitable form for topical, oral, rectal, transmucosal, transnasal, intestinal, enteral and parenteral administrations.

In a preferred embodiment, the composition is a composition for topic use. In a preferred embodiment, the composition is a composition for topic use which is administered topically to a part of the skin such as, e.g., to the face, scalp (including the hair), or part of a body. Skin may be all types of skin, including compromised skin, dry skin, healthy skin, normal skin and oily skin.

As indicated above, the galactinol of the present invention may be used for any purpose. Preferably, it is used for a cosmetic and/or pharmaceutical purpose.

Accordingly, a further aspect of the present invention relates to a cosmetic use of a composition comprising (or consisting of) 3-O-α-D-galactopyranosyl-D-*myo-*inositol defined above for maintaining and/or restoring a healthy skin through the stimulation of its own intrinsic defense mechanisms, for inducing the production of the extracellular matrix components in skin cells, for stimulating the growth factors production, for maintaining and/or reinforcing the skin microbiota; more generally for improving the global skin homeostasis and the global skin appearance.

Surprisingly, the composition of the invention is enabling to improve skin hydration, the skin appearance, to stimulate the production of the ECM components, to induce the cell's intrinsic antioxidant defense mechanisms and to activate the intrinsic cell machinery responsible for both DNA and protein protection and repair. Hence, accomplishing these functions, the global appearance of the skin may be improved and protected.

In a preferred embodiment, the intrinsic defense mechanisms may be selected from the antioxidant machinery, the protection of proteome, DNA repair enzymes, production of growth factors, or two or more thereof.

In a preferred embodiment, the composition of the invention is for use for:
a. detoxifying skin cells through the stimulation of the antioxidant defense machinery,
b. protecting skin cell's proteome and skin barrier function through the stimulation of the repair proteins,
c. protecting skin cell's DNA through the stimulation of the DNA repair genes,
d. protecting skin cells from exposure to external stress factors selected from UV irradiations and environmental stress factors,
e. promoting skin firmness and elasticity through the stimulation of the extracellular matrix genes and growth factors,
f. promoting the skin hydration through the production of hyaluronic acid and proteoglycans,
g. improving skin microrelief,
h. reducing skin's wrinkles,
i. a combination of two or more thereof.

In a preferred embodiment, the use is for:
- improving antioxidant and detoxification properties of the skin microbiota,
- improving host and microbiota metabolisms,
- preventing the skin inflammation by modulating immune response elements,
- inhibiting the fungal pathogenicity by downregulating its filamentous conversion,
- improving proteostasis in host and skin microbiota.

Accordingly, improving proteostasis in host and/or skin microbiota may comprise one or more of the following functions:
- improving ribosomal assembly,
- improving proteins transport,
- improving polypeptides degradation,
- improving proteins ubiquitination and subsequent degradation,
- proteins and amino-acids synthesis processes,
- improving chaperonins and folding process,
- improving glycoproteins synthesis and quality control,
- reducing the endoplasmic reticulum stress.

According to an embodiment, the use is for:
- improving the skin barrier function,
- reinforcing the extracellular matrix organization and its structure.

As used herein, the term "metaproteomic" may be understood as the proteomic analysis of a mixture of species and searching the mass spectrometer (MS) data with a compilation of protein sequence databases that represent at least some of the species in the mixture. The mixture may contain more than microbial species colonizing a mammalian host organism, for example, it may include host proteins. Indeed, metaproteomic allows accessing to activated functions at the human, bacteria, and fungi level, whereas metagenomics does not. As used herein, "host" means human body, preferably the skin.

As used herein, "proteostasis" may be understood as a dynamic regulation of a balanced, functional proteome. The proteostasis network includes competing and integrated biological pathways within cells that control the biogenesis, folding, trafficking, and degradation of proteins present within and outside the cell.

In a preferred embodiment, the use of the composition of the invention is for improving the skin structure, for delaying and/or preventing the apparition of wrinkles, for improving the skin hydration, for treating the loss of the mechanical properties in the skin, particularly for improving the skin firmness, smoothness, thickness, tone, glow, texture and elasticity, for preserving and/or for restoring the skin barrier function, for treating and/or preventing the imperfections of the skin, for treating and/or preventing the skin damages, for preventing or treating the hair greying, the hair loss, for promoting the hair growth, the hair density, or for two or more thereof.

Advantageously, it was observed that the composition of the present invention is enabling to improve the general appearance of the skin by acting at different levels, for example at the extracellular matrix level, at the protein and DNA levels by ensuring their protection.

As used herein, the term "global appearance" of the skin and its annexes may be understood in the broadest sense as the global status (morphologic and physiologic) of the skin.

As used herein, the term "healthy skin" may be understood in the broadest sense as capabilities of the skin to self-defend and to self-repair in order to avoid excessive inflammation, aberrant pigmentation, poor barrier function and deterioration of the extracellular matrix components.

As used herein, the term "damage" may be understood in the broadest sense as any perturbation or dysfunction in the skin and its cells, particularly in (otherwise) healthy skin. As used herein, damages may be induced by internal aggressions (aging, one or more genetic factors, one or more hormones) and external stress factors such as light exposure (e.g. exposure to ultra-violet (UV) light, exposure visible light and/or exposure to infra-red light, exposure to particular pollution, exposure to one or more pathogens and/or allergens (including natural and chemicals products), exposure to environmental stress factors (fluctuating temperature: warming and humidity), and/or exposure to mechanical constraints (including such due to daily habits such as shaving, gloves, and/or wearing masks).

As used herein, the term "annexe" may be understood in the broadest sense as it can include the hair and the hair follicles.

Galactinol or a composition comprising thereof according to any of the embodiments of the present invention may have a local and/or a systemic effect. In a preferred embodiment, when administered locally and/or topically, it (mainly or completely) has a local effect.

In a preferred embodiment, the use is for skin care, preferably wherein the use is for ameliorating the global appearance of any type of skin, including scalp.

Stimulating the expression of the ECM genes may be achieved by acting on its different components. In other words, the composition of the present invention is enabling to promote and/or stimulate the synthesis of the ECM components.

Preferably, the stimulation of the ECM genes may result in the promotion of the synthesis of the proteins selected from collagen, procollagen, elastin, fibulin, fibrillin, fibronectin and laminin or a mixture thereof. Macromolecules may be proteoglycans selected from decorin, perlecan, lumican, syndecan and versican or a mixture thereof; and/or non-sulfated glycosaminoglycans (e.g. hyaluronic acid). For example, it is known that decorin is the most highly expressed proteoglycan on the human skin, moreover its size and content decrease with aging. In an embodiment, stimulating the synthesis of hyaluronic acid may be reached by promoting the expression of the hyaluronic acid synthase 2 (HAS2).

Advantageously, targeting the ECM by acting on one or more of its components, the composition of the invention is enabling to improve the skin mechanobiology in its entirety. Indeed, improving the skin mechanobiology may plausibly lead to the prevention of the apparition of fine lines and wrinkles, the improvement of the smoothness, firmness, tone, elasticity, thickness, texture and its resilience, the improvement of the skin barrier function. It is agreed that increasing and/or stimulating the production of hyaluronic acid and proteoglycans (e.g. LUM, SDC, VCAN) leads to the improvement of the skin hydration. Moreover, these latter play a crucial role in participating in the mechanical strength of the skin. Additionally, as the production of ECM components (e.g. COL, FBN, FN, ELN and FBLN) has been increased by the composition of the invention, this may plausibly lead to the improvement of the mechanical strength of the skin.

Stimulating the expression of antioxidant defense genes may be reached by promoting the expression of the enzymes selected from superoxide dismutase 1 (SOD1), NAD(H)dehydrogenase quinone 1 (NQO1), heme oxygenase 1 (HMOX1), glutathione peroxidase 1 (GPX1), glucose-6-phosphate dehydrogenase (G6PD), or two or more. Consequently, the composition of the invention may plausibly be used for antioxidant and/or anti-free radical treatment.

Consequently, by acting at the ECM and antioxidant levels, the composition of the invention is enabling for delaying and/or preventing the signs of skin aging and for allowing the obtention of a healthy and younger skin.

Stimulating the expression of the protein repair genes may be reached by promoting the synthesis of the transforming TGFβ1, nerve growth factor receptor (NGFR), NGF, HSPA1A, HSP90AA, HSP27, or two or more thereof. Consequently, the composition of the invention may plausibly be used for protecting the skin against aggression caused by the environmental stress factors such as heat, humidity, fine particles, radiations (e.g. UV, infra-red). Moreover, promoting the production of HSP27 participates in the latest keratinocyte's differentiation step, thus in improving the skin barrier function. Consequently, targeting these proteins may plausibly lead to the improvement of the skin homeostasis, in particular skin hydration and preserving epidermis integrity.

Stimulating the expression of the DNA repair genes may be reached by promoting the synthesis of PCNA, MKi-67, GADD45A, or two or more thereof. Targeting these group of genes allows to maintain the genetic integrity of cells, and consequently the cell functions. Therefore, the composition of the invention may plausibly be used in protecting and/or limiting the senescence, the aging process, and the apoptosis of the skin cells.

It will be understood that the definitions and preferred embodiments made in the context of the composition comprising (or consisting of) galactinol of the present invention *mutatis mutandis* apply to the (cosmetic) use thereof.

The present invention also relates to 3-O-α-D-galactopyranosyl-D-*myo*-inositol as defined above or a cosmetic or pharmaceutic composition as defined above for use as a medicament.

The present invention also relates to a composition comprising 3-O-α-D-galactopyranosyl-D-*myo*-inositol as an active ingredient for use in a non-therapeutic method for maintaining and/or for improving the global appearance of the skin and its annexes, wherein the composition is for maintaining or ameliorating the skin homeostasis, for maintaining a healthy skin, for improving the skin firmness and/or elasticity, for protecting skin against external stress factors, for improving skin relief, for improving the skin hydration and skin barrier function, for preventing and/or delaying the signs of skin aging; and wherein, for already aged skin, the composition is for preserving and reinforcing the mobilization of its microbiota resources.

Throughout this specification and the claims, unless the context requires otherwise, the open-worded terms such as "comprise", "contain", "include", etc., and variations such as "comprises", "contains", "includes", "comprising", "containing", "including", etc., maybe understood to imply the inclusion of a stated component, member, integer or step or group of components, members, integers or steps, but not the exclusion of any other components.

The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually as well as in combination with each other recited herein. All methods and procedural steps, including product-by-process steps described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

It should be understood that the aspects and embodiments may be combined in any manner and in any number to additional embodiments. Any permutations and combinations of all described elements in this application should be considered disclosed by the description of the present application unless the context indicates otherwise.

Unless defined otherwise, all technical and scientific terms used herein have the same meaning as commonly understood by a person skilled in the art. Although the methods and materials described herein are preferred, other methods and materials similar or equivalent to those described herein can be used in the practice or testing of the present invention as well.

All documents cited or referenced herein are hereby incorporated by reference and may be employed in the practice of the invention. More specifically, all referenced documents are incorporated by reference to the same extent as if each individual document was specifically and individually indicated to be incorporated by reference.

The following Examples and Figures are intended to illustrate further embodiments of the present invention without limiting its scope.

### Examples

1. Preparation of 3-O-α-D-galactopyranosyl-D-*myo*-inositol For the following, the 3-O-α-D-galactopyranosyl-D-*myo*-inositol is named galactinol.

In a 1 L reactor, 148 g of raffinose pentahydrate was dissolved at 45°C and stirred at 300 rpm in 660 mL of 100 mM Sodium Phosphate buffer at pH 5.0±1. 8 mL of β-fructofuranosidase (Invertin^{™} by Merck; 2.4 KU/mL) was added and the reaction mixture was allowed to stir at 45°C for 2 hours. High performance liquid chromatography (HPLC) analysis showed complete conversion of raffinose to melibiose after 1 hour.

180 g of *myo*-inositol was added, and pH was adjusted to 7.0±1 by adding sodium hydroxide. After complete solubilization of *myo*-inositol, 1,85 g of α-galactosidase (Sumizyme^{™} AGS; 30 KU/g) was added and the reaction was allowed to stir at 45°C while taking samples each hour. After 5 h, HPLC analysis showed a galactinol concentration higher than 50 mM and the reaction was quenched by heating the reaction mixture at 75°C for 3 to 10 minutes.

After cooling down to 30°C, 300 mg of MgCl2.6 H20 and 25 g of freeze-dried Baker's Yeast were added, and the resulting mixture was allowed to stir at 30°C for 40 hours. The reaction mixture was centrifuged, and the supernatant was concentrated on the rotary evaporator at 70°C under reduced pressure. The concentrated solution was allowed to stir for 1 hour at 70°C for inducing crystallisation. The temperature of the suspension was allowed to reach room temperature over a period of 4 hours while hydroethanolic was added over the same period as anti-solvent. After stirring overnight at room temperature, the suspension was stirred for 2 hours at 0°C after which the suspension was filtered. The crystals were washed with cold hydroethanolic and the filtrate and washing liquid were both analysed by HPLC. The combined filtrate and washing liquid were concentrated until the ethanol content was below 2500 ppm and the resulting solution was passed over hydrophobic resin (Dowex^{™} Optipore L-490).

The decolored solution was analysed by HPLC and water and glycerol were added to obtain 80/20 glycerol/water (v/v) solution containing galactinol.

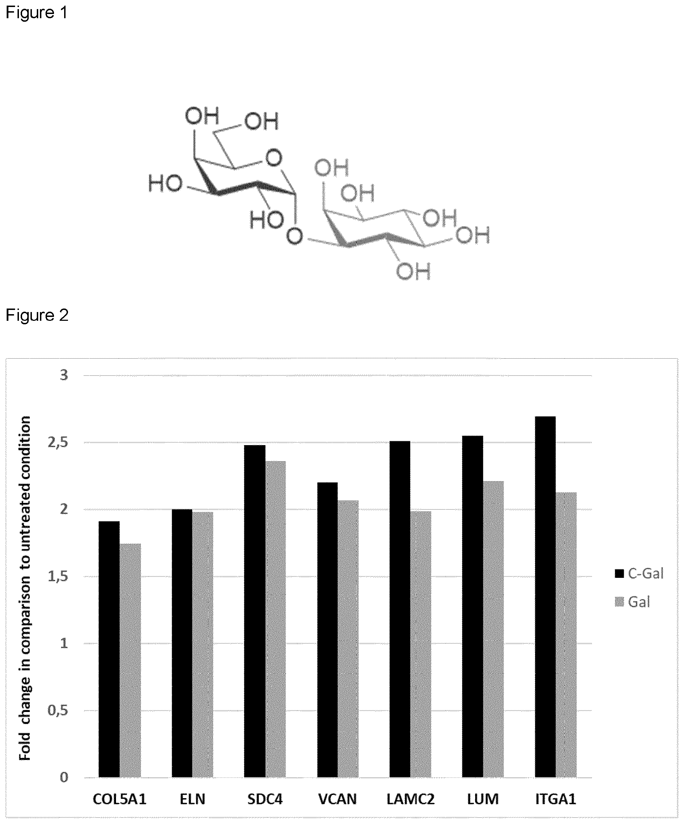

Figure 1 shows the chemical structure of the 3-O-α-D-galactopyranosyl-D-*myo-*inositol (galactinol) of the invention. It is composed of a *myo*-inositol moiety and a galactose moiety.

HPLC chromatogram confirmed that the composition of the invention comprises galactinol (data not shown). For performing this analysis, samples were analyzed by HPLC equipped with an ELSD detector using a Luna Omeag Sugar column (250 x 4.6 mm 3 µm) under isocratic conditions (75% acetonitrile; 25% water) at 1 mL/min at 30°C. Moreover, 1H and 13C NMR analysis confirmed that the compound that elutes at retention time (RT) at 25.5 minutes corresponds to 3-O-α-D-galactopyranosyl-D-myo-inositol and the one at RT 26.5 minutes is an isomer (a-O-D- linkage) of galactinol.

### 2. In vitro results:

### 2.1 Transcriptomic studies:

In the following studies, the capabilities of the composition of the present invention to improve skin hydration, the skin appearance, to stimulate the production of the ECM components, to induce the cell's intrinsic antioxidant defense mechanisms and to induce the intrinsic cell machinery responsible for both DNA and protein protection and repair were demonstrated in normal human dermal fibroblasts (NHDF) using RT-qPCR technology (Taqman).

For the following, the galactinol and a composition comprising thereof, according the embodiments of the present invention, will be named Gal and C-Gal respectively.

### 2.1.1 Methods:

The mRNA expression was quantified after 24h of NHDF treatment with both Gal and C-Gal.

mRNA extraction was realized according to the provider recommendations (Qiagen). Briefly, lysis buffer RLT-Plus was added on the cell pellet and the homogenous lysate was transferred in a column. The eluate was mixed with one volume 70° ethanol and was transferred in a pink « RNeasy » column. The column was washed with buffer. Elution was realized with RNase-free water and the RNA quantitation was done by D.O. 260nm, 280nm, 320nm measurements (Biotek, Cytation3 & Take3 Plate) with "Nucleic Acid Quantification" software application. mRNA quality was checked using a fragment analyser (Advanced Analytical). Then, mRNA reverse transcription allowing the obtention of cDNA templates was performed on a thermal cycler (Bio-Rad). cDNA templates were used to perform the qPCR. For the transcriptomic study, it was used Biorad predesigned plates for ECM components, antioxidants genes, skin homeostasis. Then, the data generated allowed the automatic calculation using Bio-Rad CFX manager of mRNA expression fold change and p values in comparison to untreated condition.

All data are statistically significant compared to control condition (p value < 0.05).

### 2.1.2 Results:

### 2.1.2.1 Stimulation of skin's key ECM components

Figure 2 demonstrated the modulation of ECM genes and proteins expressions of the NHDF treated with both Gal and C-Gal. Many ECM components such as COL5A1, ELN, SDC4, VCAN, LAMC2, LUM and Integrin α-1 (ITGA1) which are known to confer tensile and mechanical strength, resilience, elasticity and hydration. These experimental results demonstrated that the ECM components were targeted by Gal and C-Gal, confirming its ability to improve the skin mechanobiology in its entirety. Therefore, both Gal and C-Gal may plausibly be used in preventing of the apparition of fine lines and wrinkles, improving the smoothness, firmness, thickness, tone, elasticity, texture, and resilience of the skin, improving in addition the skin hydration. In other words, the composition of the invention is usable as an active ingredient for improving the global skin appearance and its condition.

2.1.2.2 Stimulation of skin intrinsic antioxidant defense machinery As shown in Figure 3, the antioxidant genes in NHDF were modulated by the treatment with Gal and C-Gal. In brief, NQO1 is a detoxifying enzyme via quinone/hydroquinone system. HMOX1 is implicated in the cellular protection against oxidative stress, GPX1 is known as one of the most important antioxidant enzymes in humans in the detoxification of hydrogen peroxide (H₂O₂) and G6PD is responsible of supplying energy to cells by maintaining the level of NADPH, which in turn maintain the supply of glutathione in the cells that is used to mop up free radicals that cause oxidative stress.

### 2.1.2.3 Stimulation of genes involved in the global maintenance of skin homeostasis, in particular the growth factors, the heat shock proteins, the DNA repair and the cell proliferation markers

As shown in Figure 4, it was demonstrated in NHDF among the modulated genes an upregulation of growth factors such as TGF Beta 1 and NGF. TGFβ1 is involved in the control of cell growth, cell proliferation, cell differentiation and apoptosis. NGF is involved in the proliferation cycle of cells. Additionally, it was observed an upregulation of heat shock proteins mainly HSPA1A and HSP90AA. HSPA1 is known to facilitate the proper folding of newly and misfolded proteins, to stabilize and degrade altered proteins. HSP90AA promotes the maturation, structural maintenance and proper regulation of proteins involved for instance in cycle control. Then, it was found an upregulation of mRNA expressions of PCNA, MKi67 and GADD45A by Gal and C-Gal. These genes encode for DNA repair and cell proliferation proteins. PCNA acts as a processivity factor for DNA polymerase delta and is essential for replication. MKi-67 is a marker of cell proliferation. GADD45A gene is a member of a group of genes whose transcript levels are increased following stressful growth arrest conditions and treatment with DNA-damaging agents.

These experimental results demonstrated the ability of both Gal and C-Gal:
- to improve the cell proliferation, to limit and/or prevent the cell senescence, hence more generally to prevent and/or to delay the signs of skin aging,
- to improve the skin homeostasis and therefore to protect skin against various stresses responsible for an alteration of skin homeostasis,
- to protect against protein (e.g. enzymes, nuclear factors) alterations,
- to repair DNA oxidative damages (e.g. due to the external aggressions such as UV rays, microparticles, etc.) and,
- to improve the skin remodeling, more generally the global appearance of the skin and its condition that is consistent with the results obtained on the ECM as detailed above.

Consequently, the composition of the invention may be usable in preventing of the apparition of fine lines and wrinkles, in improving the skin smoothness, in preventing the hair loss, in improving the hair density.

### 2.2 Assessment of skin's cells protection properties using cell morphology observations

In the following studies, the capabilities of Gal and C-Gal to protect skin cells (NHEK and NHDF) against various stress agents such as UV irradiations (UVB and UVA), or environmental factors (humidity and temperature) were assessed.

### 2.2.1 Methods:

Briefly, using a bright-field imaging microscope, it was studied the effect of the UVB irradiation on NHEK's morphology in various experimental conditions in presence or not of Gal and C-Gal:
- "Preventive" condition: Gal or C-Gal was applied on NHEK during 24 hours before UVB irradiation (30mJ/cm²), and the morphology of the cells was observed after 24 hours,
- "Curative" condition: Gal or C-Gal was applied after UVB irradiation, and the morphology of the cells was observed after 24 hours,
- Combined condition "preventive and curative": Gal or C-Gal was applied before and after UVB irradiation and the morphology of the cells was observed after 24 hours.

The same experiments as described above were repeated by using UVA irradiation on NHDF in the following condition:
- "Curative" condition: Gal or C-Gal was applied after UVA irradiation (10J/cm²), and the morphology of the cells were observed after 24 hours.

Finally, the effect of environmental stress on cell morphology was studied in NHEK treated with and without Gal or C-Gal (using a bright-field imaging microscope). The environmental stress applied were either a heat stress (increase in temperature from 37°C to 44°C during 30 min), or an hydric stress "modulation of relative humidity from 90% to 45%" or a combination of both stresses "heat plus hydric stresses".

### 2.2.2 Results (data not shown)

It was observed in NHEK and NHDF cells a deleterious effect of UVB, UVA and environmental stress factors (heat and humidity). Indeed, in stress condition, in UVA and UVB conditions less cells are present (suggesting the death of the cells under these stress conditions), and cell's morphology was clearly altered. Indeed, healthy keratinocytes have a polygonal shape while in stress condition they appear more elongated (like fibroblasts). In contrast, in the environmental stress condition, any decrease in the cell numbers was observed. In parallel, a strong alteration of cells morphology appeared suggesting an alteration of their homeostasis. Hence, the composition of the present invention is suitable to protect the cells from UVB, UVA and environmental damages. Indeed, in comparison to stress condition alone more cells are present in the stress treated condition with Gal and C-Gal. Moreover, a quite regular and expected cells morphology was observed in the treated group.

### 2.3 Assessment of skin's cells protection properties of the composition through the stimulation of HSP27 protein expression

### 2.3.1 Method:

NHDF were treated with Gal or C-Gal for 24h (treated cells). Then, cells were fixed for 30 minutes and immuno-stained with a primary antibody HSP27 (ab62339, Abcam) revealed with a secondary antibody conjugated with Alexa fluor 568 (A1101, Thermofisher). Pictures were realized with a Nikon microscope (Eclipse Ti2). The quantification of HSP27 was measured by image analysis of fluorescence intensity per cell using Image J software. The statistical analysis (Dunnett) was realized with Minitab software (*p < 0.05 compared to control). Results were obtained by performing three independent assays.

### 2.3.2 Results

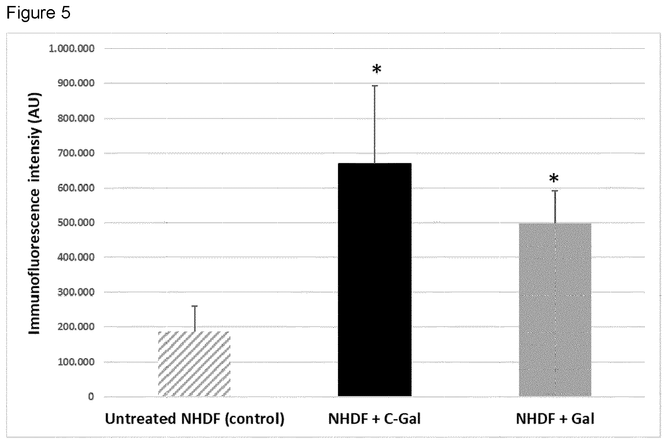

In Figure 5, it was observed in basal condition (i.e. non stressed condition) a clear stimulation of HSP27 expression in treated cells. This result suggested that the Gal or C-Gal can plausibly provide a preventive and protective effect to skin cells.

### 2.4 Assessment of skin hydration properties

In order to investigate whether the composition of the present invention is capable to improve the skin hydration, it was assessed its effect on the production of hyaluronic acid (HA) by NHEK cells.

### 2.4.1 Methods:

NHEK were cultured in medium DERMALIFE^{™} K and penicillin/streptomycin in a humidified atmosphere containing 5% CO₂ in air at 37°C for 24 hours.

After 24 hours, cell culture medium was substituted by 10 µL of C-Gal or by EGF (Gibco^{™}, ref: 2116096) at 10⁻⁸M final, as a positive control, diluted with 90 µL of culture medium per well and then incubated in a humidified atmosphere containing 5% CO₂ in air at 37°C for 48 hours. Then, the supernatants were collected and centrifuged afterwards in order to dose the hyaluronic acid produced by the cells. The total content in protein was quantified using the Bradford method in order to normalize for each condition the hyaluronic acid quantified. Finally, the hyaluronic acid dosage was performed by ELISA technic according to the supplier recommendations (Hyaluronan DHYALO kit from R&D System).

All data were expressed as mean ± standard deviations. The statistical significance of the data was determined by two-way analysis of variance with Minitab software (*p < 0.05 compared to control). Results were obtained by performing three independent assays.

### 2.4.2 Results

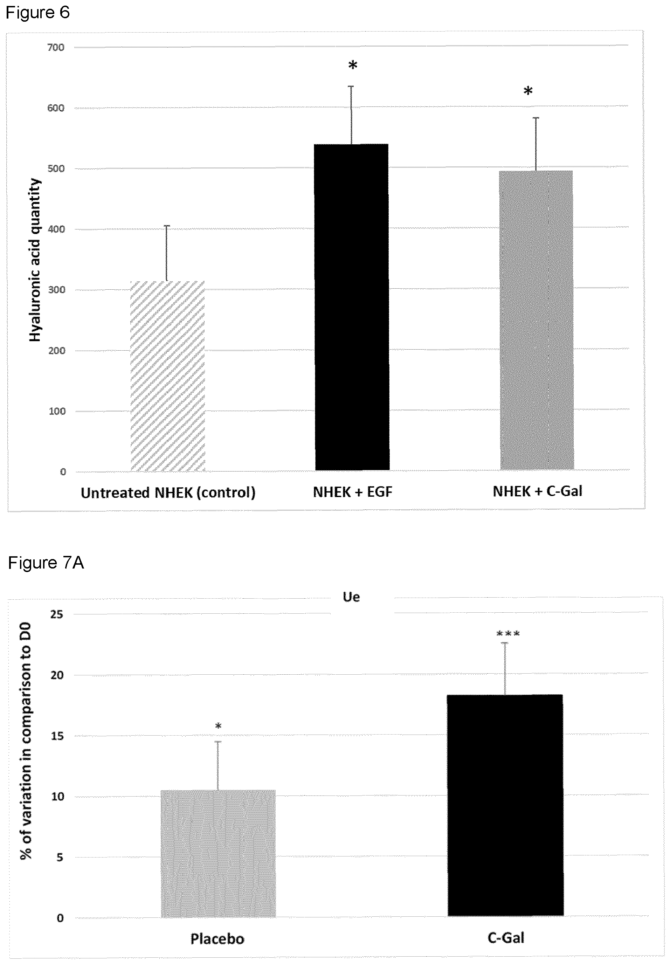

Figure 6 shows the ability of C-Gal to promote the synthesis of HA by NHEK. Consequently, the skin hydration is improved thanks to the increased production of HA within skin cells. In addition, RT-QPCR analysis showed that C-Gal significantly stimulated the mRNA expression of HAS2 (hyaluronan synthase 2, fold change x 2.2) an enzyme responsible for HA synthesis.

### 3. Clinical study:

### 3.1 Assessment of the properties of the composition of the invention to improve human skin biomechanical properties

### 3.1.1 Methods:

A randomized double-blind placebo controlled clinical trial was conducted. The study was conducted in accordance with the principle of the Declaration of Helsinki and the guidelines of the International Conference on Harmonization Good Clinical Practice, as applicable to a non-drug study.

20 Caucasian females were enrolled in this clinical study which lasted 28 days. The volunteers had an age range of 50-60 years and were presenting wrinkles, as well as a dull and uneven complexion due to daily photo-exposition. Subjects applied the placebo and a formulation containing the composition of the invention at 0.3% on one half of their face.

**Table 1: cream containing the composition of the invention at 0.3%, and placebo**

| Composition of cream in % weight | Placebo | C-Gal (0.3%) |
|---|---|---|
| Ammonium Acryloyldimethyltaurate/VP Copolymer | 0.8 | 0.8 |
| Dicaprylyl Ether | 1 | 1 |
| Buffer (Citric acid and sodium citrate) | 0.1 | 0.1 |
| Phenoxyethanol/ Methylparaben/Ethylparaben | 1.13 | 1.13 |
| Fragrance | 0.03 | 0.03 |
| C-Gal | 0.00 | 0.3 |
| Water | 96.94 | 96.64 |

The parameters were measured by the means of AEVA-HE device (by EOTECH) and a cutometer (courage and Khazaka). AEVA-HE device was deployed to take high 2D and 3D resolution pictures on wrinkle volume. The cutometer allows the determination of Ue and Ur parameters, which are two parameters measuring respectively skin elasticity and skin firmness.

### 3.1.2 Results:

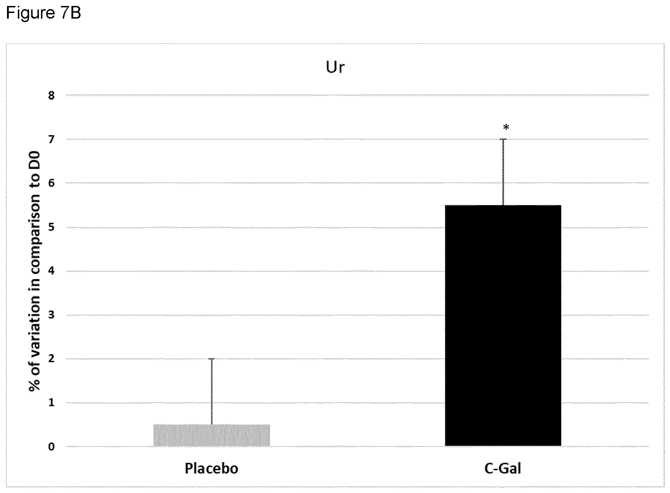

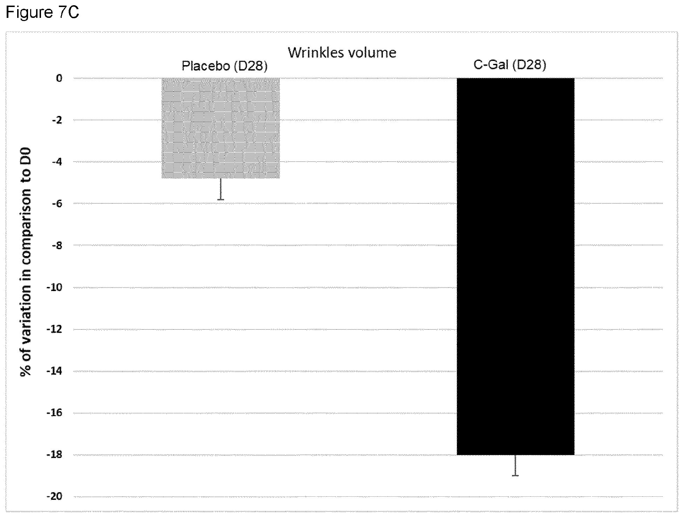

Figure 7 (A and B) shows a significant visible improvement of skin biomechanical properties after 28 days of use of the composition of the invention, compared to the start day (D0). Indeed, both parameters Ue (skin elasticity) and Ur (skin firmness) increase after 28 days following the application of the cream in table 1. These parameters are known to be strongly decreased with chronological aging but also during photoaging. Thus, it can be postulated that the composition of the invention is able to improve the elasticity, the firmness and the smooth of the skin, more generally the global appearance of the skin.

All data were expressed as mean ± standard deviations. The statistical significance of the data was determined by two-way analysis of variance with Minitab software (***p<0.001 compared to D0; **p<0.01 compared to D0; *p < 0.05 compared to D0). Results were obtained by performing three independent assays.

Moreover, the measure of wrinkles volumes before, meaning at D0, and after 28 days of treatment by the composition of the invention showed a significant decrease in the wrinkles' volume in treated group (Figure 7C). This result demonstrated the ability of the composition of the invention in significantly reducing the wrinkles.

### 4. Study of metaproteomic effects of the composition of the invention:

### 4.1 Materials and methods:

Twenty volunteers were recruited for the metaproteomics analysis. Samples were collected before (day 0) and after (day 28) of treatment with C-Gal on one side of the face and placebo on the other side.

The aim of the metaproteomic study was to investigate simultaneously proteins from host (Human body) and its microbiome (bacteria and fungi). A bioinformatic tool called HolXplore^{™}, developed by the company Phylogen S.A (France) was used for taxonomic and functional analysis. Analysis were conducted according to the supplier's recommendations. It comes to a data processing module for host and microbiome outputs of Omics comparison studies. It comprises a taxonomic analysis of the diversity combined with different experimental conditions, a functional analysis in parallel for bacteria, fungi, and homo sapiens taxon's, the correlation between different function and/or taxon.

Briefly, proteins, whatever their taxonomic origin, were extracted from skin swabs and digested. Peptides extracts were labeled and analyzed by MS/MS fragmentation and nano-LC HRAM MS technology (nano chromatography coupled with High Resolution Accurate Mass Spectrometer).

Then, proteins were identified using SEQUEST-HT algorithm against all bacteria, fungi and human reference proteomes mined from UniProt KB, along with sequences of most common experimental contaminants in LC-MS/MS, except sequences also found in human reference proteome.

The taxonomic analysis was focused on microbiome composition in terms of taxa abundances. It allows the taxonomic assignment of each protein according to their peptide taxonomy.

Identified taxa were gathered in three different taxonomic groups: host (human), bacteria and fungi. The diversity analysis then highlighted differences in microbiome compositions and diversity between samples groups. The abundance of each taxon was calculated as the sum of associated proteins abundances. Those abundances were used as input data to measure "alpha-diversity" and "beta-diversity" using respectively "diversity" and "vegdist" functions of "vegan" R package:
- alpha-diversity measures intra-sample diversity
- beta-diversity measures inter-samples diversity and samples separation according to their microbiome composition.

The functional analysis was focused on functions of identified proteins. Each protein was functionally annotated, and an enrichment analysis is performed to highlight modified biological processes. A large part of microbial proteins lacks functional annotation. For this, protein sequences (recovered from Uniprot and Uniparc databases) were submitted to the bioinformatic known tools EggNOG mapper to associate each protein to its closest annotated ortholog. The functional annotation of orthologs was used for further analysis.

### 4.2 Results:

### 4.2.1 Form taxonomic analysis:

The analysis of beta-diversity did not highlight a separation of samples according to the two treatments (placebo versus C-Gal). In contrast, the alpha-diversity analysis highlighted a significant difference in samples diversity between the two treatments, with a greater diversity for the active group C-Gal without perturbation of the microbiome composition. So that the composition of the invention is safe for skin microbiota, and then can be considered as "microbiota-friendly". Interestingly, thanks to the taxonomic analysis, it was observed an upregulation of the genera *Amycolatopsis* bacteria which is known for its secondary metabolites exerting antimicrobial, anti-cancer, and antioxidant activities.

### 4.2.2 From functional analysis:

It demonstrated that C-Gal modulated:
- antioxidant and detoxification properties of the skin microbiota treated with C-Gal: abundances of proteins associated to oxidative stress were modulated in fungi and bacteria. Peroxiredoxins are antioxidant enzymes that also control peroxide levels and mediate the associated signaling events, notably in association with Thioredoxin. Three Peroxiredoxin and two Thioredoxins were regulated. Some proteins potentially involved in antioxidant response were also regulated as SOD, an uncharacterized protein ortholog of Glycine cleavage system H protein-3 (GDH3) which plays a central role in maintenance of the redox balance, and luciferase proteins involved in the oxidation of long-chain aldehydes. Finally, proteins involved in other types of cellular stress as formaldehyde detoxification and genotoxic stress from fungal origins were also regulated.
- host and microbiota metabolisms: energy metabolism allows cells to produce energy from various sources. Glucose in one of the major energy sources. Results clearly highlighted an upregulation of glucose (mainly composed by GAPDH proteins) and lipids metabolism in bacteria and fungi, and lipids metabolism in host (mainly associated to Perilipin-5 (PLIN5), a protein involved in lipids storage). C-Gal upregulated a set of proteins involved in cell wall components production as peptidoglycans, lipopolysaccharides and mycolic acids.
- anti-inflammatory properties: immunity allows host to control its microbiome to avoid infections and dysbiosis, but a chronic inflammation can lead to skin dysfunctions and diseases. For the immune component, host regulated proteins suggested a decrease of inflammation, principally by the downregulation of Complement C8 beta chain (C8B: a component of membrane attack complex) and upregulation of negative regulators (CD81, Receptor-type tyrosine-protein phosphatase epsilon (PTPRE), and Ras-related protein Rab-7b (RAB7B)) of inflammation signaling. CD81 increased Breakpoint cluster region protein (BCR) signaling pathway, PTPRE inhibits cytokines production and degranulation, RAB7B negatively regulates Toll-like receptors (TLR) pathways and subsequent inflammation. Moreover, C-Gal modulated the Cathelicidin antimicrobial peptide which is a direct actor on microbial population.
- inhibition of fungal pathogenicity: microbiota proteins are also able to impact host functions. Some commensal fungi are able to switch from yeast to filamentous forms (morphogenesis essential for their potential pathogenicity). Filamentous forms can invade keratinized epitheliums leading to the disruption of epidermal barrier function and finally triggers diseases. Proteins implicated in filamentous growth were regulated orthologs of ELM1, ASC1 and the transcription factor RBF1 which were able to inhibit filamentous growth. Those results suggested a downregulation of filamentous form, leading to a decreased invasion of epithelial barrier so the preservation of a better skin health is ensured.

- proteins regulation pathways also called "proteostasis": a part of them are ribosomal proteins, from bacterial or human origins. Proteins involved in proteins transport in host was also regulated, as peptidases, involved in polypeptides degradation and enzymes involved in proteins ubiquitination and subsequent degradation. Moreover, proteins were folded in the endoplasmic reticulum. Accumulation of misfolded or unfolded proteins leads to endoplasmic reticulum stress (ER stress), closely related to oxidative stress. Chaperonins are essential for correct proteins folding, and some of them, mostly from bacterial origins, were regulated. Three Peptidyl proline isomerases (PPlases), which are accelerators of this folding process, were upregulated. The downregulation of Reticulocalbin-1 supported the hypothesis of a decrease of ER-stress because of its role in ER-stress induced apoptosis.
- C-Gal showed an improvement of skin barrier function: besides inflammation and fungal invasion, various parameters may affect skin barrier integrity, as cell-cell and cell-matrix adhesion of host cells. The p53 apoptosis effector related to PMP-22 (PERP) is a component of desmosome, and increased desmosome assembly is an indication of stronger epithelial barrier and skin integrity. Finally, precursors of the cornified envelope of the stratum corneum were also regulated.
- reinforcement of ECM organization and structure: Matrilysin is an enzyme able to degrade ECM and its downregulation could be linked to stronger ECM structure or decrease tissue remodeling. Moreover, C-Gal stimulated the glycosaminoglycans (GAGs) synthesis through the upregulation of the crucial enzyme UDP-glucuronic acid decarboxylase 1 involved in GAGs synthesis.
- The experimental metaproteomics results obtained with the composition of the invention confirmed, in combination with the above experiments, its ability in enhancing the global appearance of the skin, for example, in rendering the skin healthier through the preservation and reinforcing the mobilization of its microbiota resources. Hence, the composition of the invention can plausibly be used for obtaining the both "pre-aging" aging and "pro-aging" effects.

## Claims

1. A composition comprising or consisting of 3-O-α-D-galactopyranosyl-D-myo-inositol as an active ingredient.

2. A composition according to claim 1, wherein it comprises a galactinol isomer having α-linkage other than the 3-α-D linkage selected from 1-O-α-D-, 2-O-α-D-, 4-O-α-D-, 5-O-α-D-, or 6-O-α-D-galactopyranosyl-D-*myo*-inositol.

3. A composition according to any of the claims 1 or 2, wherein the molar ratio between the 3-O-α-D-galactopyranosyl-D-*myo*-inositol:galactinol isomer having α-linkage other than the 3-α-D linkage within the composition is approximatively from 3:1, preferably 2:1, in particular 1.5:1

4. A composition according to any of the claims 1 to 3, wherein the mixture comprising 3-O-α-D-galactopyranosyl-D-*myo*-inositol and galactinol isomer represents 20% by weight, preferably 25% by weight, in particular 30% by weight relative to the total weight of the composition.

5. A composition according to any of the claims 1 to 4, wherein it further comprises melibiose or *myo*-inositol or monosaccharide or a mixture thereof, wherein monosaccharide being selected from galactose, glucose, fructose, or a mixture thereof.

6. A composition according to any of the claims 1 to 5, wherein it comprises:
- melibiose 0 to 25 weight %,
- myo-inositol 0 to 40 weight %,
- monosaccharides 0 to 30weight %
over the total weight of the composition.

7. A process for biocatalytic production of a galactinol having the formula 3-O-α-D-galactopyranosyl-D-*myo*-inositol comprising the steps:
A1) Providing melibiose, or incubating raffinose with β-fructofuranosidase, at pH of 3 to 8, for obtaining melibiose; and incubating the mixture comprising melibiose and an acceptor selected from pinitol and/or inotisol isomers comprising *myo-, chiro-, scyllo-, chiro-, neo-, allo-, epi-* and *cis*-inositol, preferably *myo*-inositol with α-galactosidase at pH of 4 to 9; or
A2) Incubating a mixture comprising an acceptor selected from pinitol and/or inotisol isomers comprising *myo-, chiro-, scyllo-, chiro-, neo-, allo-, epi-* and *cis*-inositol, preferably *myo*-inositol and raffinose and/or stachyose with α-galactosidase at pH of 3 to 9; without having conducted step A1);
B) Purifying 3-O-α-D-galactopyranosyl-D-*myo*-inositol from the mixture of compounds obtained in step A1) or in step A2).

8. A process according to the claim 7, wherein the β-fructofuranosidase is added to the raffinose for a period in the range of 15 up to 160 minutes at a temperature in the range of 20 to 60°C, preferably at 25 to 55°C, in particular at 30 to 50°C.

9. A process according to any of the claims 7 or 8, wherein the myo-inositol is added after a period from 30 up to 150 minutes after the step A1) was being started.

10. A process according to any of the clams 7 to 9, wherein the purification of the reaction mixtures obtained in step A1) or in step A2) is conducted by yeast fermentation.

11. A process according to any of the claims 7 to 10, wherein a precipitation step by hydroalcoholic solvent is further performed on the remaining reaction mixture obtained after the yeast fermentation.

12. A cosmetic or pharmaceutical composition comprising:
a. 3-O-α-D-galactopyranosyl-D-*myo*-inositol as an active ingredient; and
b. at least one further cosmetically and/or pharmaceutically acceptable ingredient other than 3-O-α-D-galactopyranosyl-D-*myo*-inositol,
wherein the composition is a composition for topical use selected from the group consisting of a solution, a suspension, an emulsion, a cream, a paste, a gel, a lotion, a powder, a soap, a surfactant-containing water, an oil, a shampooing, and a spray; and/or wherein the composition is a nutraceutical composition which is administered orally.

13. A cosmetic use of a composition comprising or consisting of 3-O-α-D-galactopyranosyl-D-*myo*-inositol according to any of the claims 1 to 6 and 12, for maintaining and/or restoring a healthy skin through the stimulation of its own intrinsic defense mechanisms, for inducing the production of the extracellular matrix components in skin cells, for stimulating the growth factors production, for maintaining and/or reinforcing the skin microbiota; more generally for improving the global skin homeostasis and the global skin appearance.

14. A cosmetic use according to claim 13, wherein the intrinsic defense mechanism is selected from the antioxidant defense machinery, the protection of proteome, DNA repair enzymes, production of growth factors, or two or more thereof.

15. A cosmetic use according to any of claims 13 or 14, wherein the use is for:
a. detoxifying cells through the stimulation of the antioxidant defense machinery,
b. protecting skin cell's proteome and skin barrier function through the stimulation of the repair proteins,
c. protecting skin cell's DNA through the stimulation of the DNA repair genes,
d. protecting skin cells from exposure to external stress factors selected from UV irradiations and environmental stress factors,
e. promoting skin firmness and elasticity through the stimulation of the extracellular matrix genes and growth factors,
f. promoting the skin hydration through the production of hyaluronic acid and proteoglycans,
g. improving skin microrelief,
h. reducing skin's wrinkles,
i. a combination of two or more thereof.

16. A cosmetic use according to claim 13, wherein the use is for:
- improving antioxidant and detoxification properties of the skin microbiota,
- improving host and microbiota metabolisms,
- preventing the skin inflammation by modulating immune response elements,
- inhibiting the fungal pathogenicity by downregulating its filamentous conversion,
- improving proteostasis in host and skin microbiota.

17. A cosmetic use according to any of claims 13 or 16, wherein improving proteosatsis in host and/or skin microbiota comprise one or more of the following functions:
- improving ribosomal assembly,
- improving protein transport,
- improving polypeptide degradation,
- improving protein ubiquitination and subsequent degradation,
- improving proteins and amino-acids synthesis processes,
- improving chaperonins and folding process,
- improving glycoproteins synthesis and quality control,
- reducing the endoplasmic reticulum stress.

18. A cosmetic use according to any of claims 13 to 17, wherein the use is for:
- improving the skin barrier function,
- reinforcing the extracellular matrix organization and its structure.

19. A cosmetic use according to any of the claims 13 to 18, wherein the use is for improving the skin structure, for delaying and/or preventing the apparition of wrinkles, for improving the skin hydration, for treating the loss of the mechanical properties in the skin, particularly for improving the skin firmness, smoothness, thickness, tone, glow, texture and elasticity, for preserving and/or for restoring the skin barrier function, for treating and/or preventing the imperfections of the skin, for treating and/or preventing the skin damages, for preventing or treating the hair greying, the hair loss, for promoting the hair growth and the hair density, or for two or more thereof.

20. A composition comprising or consisting of 3-O-α-D-galactopyranosyl-D-myo-inositol as an active ingredient for use in a non-therapeutic method for maintaining and/or for improving the global appearance of the skin and its annexes, wherein the composition is for maintaining or ameliorating the skin homeostasis, for maintaining a healthy skin, for improving the skin firmness and/or elasticity, for protecting skin against external stress factors, for improving skin relief, for improving the skin hydration and skin barrier function, for preventing and/or delaying the signs of skin aging; and wherein, for already aged skin, the composition is for preserving and reinforcing the mobilization of its microbiota resources.
